# EUROPEAN PATENT SPECIFICATION

(11) **EP 0 296 142 B1**
(45) Date of publication and mention of the grant of the patent: **28.02.1996**
(21) Application number: 88870097.8
(22) Date of filing: 27.05.1988
(51) Int. Cl.: A61N 1/16, A61N 2/08

(54) **Device to counterbalance the surrounding electromagnetic radiation**
Ausgleichsvorrichtung für die umgebende elektromagnetische Strahlung
Dispositif pour contrebalancer la radiation électromagnétique environnante

(30) Priority: 05.06.1987 BE 8700622
(43) Date of publication of application: 21.12.1988
(73) Proprietor: Deckers, François, B-1932 Sint-Stevens-Woluwe (BE)
(72) Inventor: Deckers, François, B-1932 Sint-Stevens-Woluwe (BE)
(74) Representative: Vanderperre, Robert

(56) References cited:
- EP-A- 0 144 610
- AT-B- 373 780
- DE-A- 1 900 744
- DE-A- 2 629 432
- FR-A- 1 514 401
- FR-A- 2 210 420
- FR-A- 2 371 916
- FR-A- 2 580 180

## Description

This invention concerns a device to counterbalance the harmful effects of surrounding electromagnetic radiation and in particular the radiation emitted by electric and electronic machines or apparatus with a view to preventing the users of such machines or apparatus or preventing persons standing in the vicinity of such machines from being subject to physiological disorders.

Electric and electronic machinery in general are known to emit electromagnetic radiation of various wavelengths. Howevers, in the last few years the realization has gradually dawned that the habitual users of certain electronic machines and apparatus including the appliances and electronic devices that are becoming more and more common in offices or daily home life, are subject to physiological disorders. This is the case of the users of office CRT screen monitors and television receivers. The statistics collected in a number of countries have revealed in particular that the regular operators of such electronic machines suffer systematically from such complaints as troubles with vision, dizziness, muscle aches and stiffness, headache, and irritability.

Although the exact causes of these complaints are still not completely understood and explained, various specialists who have observed and analyzed these phenomena attribute them chiefly to the electromagnetic radiation emitted by the machines.

The applicant has studied this problem and, based on the idea that living cells grow and develop naturally when they are in a balanced electromagnetic field, believes that the foregoing complaints and disorders result from an imbalance in the surrounding electromagnetic waves due to an excess of harmful waves and more particularly those harmful waves generated by electronic machines.

The object of this invention is a device to counterbalance the harmful radiation generated locally by a machine. This goal is achieved according to this invention by a device comprising a receptacle made of a non metallic material and at least one interactive module placed in the receptacle, each interactive module comprising two parts, a first part consisting of a permanent magnet and the second part consisting of a bar made of carbon, natural or artificial graphite, said two parts being spaced apart such that said bar is traversed by magnetic flux lines produced by said magnet. The receptacle has fixing or suspension means for the fixation or suspension of the device onto a surface of or inside a machine or for allowing the device to be hung from a necklace.

The invention is described below with the help of the accompanying drawings in which :
- Fig. 1 is a top view of an exemplary embodiment of the device according to the invention with the cover removed;
- Fig. 2 is a cross-sectional view of the device of Figure 1;
- Fig. 3 is a rough diagram showing how several of the devices of Figure 1 are arranged on a typical screen display;
- Fig. 4 depicts schematically a second exemplary embodiment of the invention.

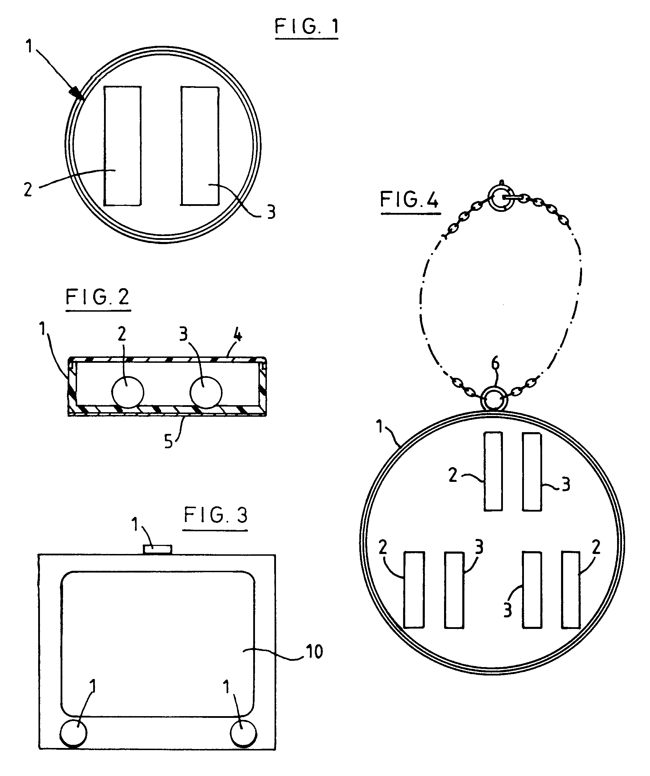

Referring to Figs. 1 and 2 there is depicted an exemplary embodiment of the device of the invention. Fig. 1 shows the device with cover removed. In a non metallic receptacle 1, for example a polystyrene box having a diameter of about 60 mm, there is placed an interactive module comprised of two spaced apart parts : a permanent magnet 2 and a bar 3 made of carbon or natural or artificial graphite. These parts have for example a length of 20 mm and are adapted in recesses provided in the bottom of the box 1. As shown in Fig. 2 the cover 4 fits sealingly the upper edge of the box. The two parts 2 and 3 are spaced apart such that the carbon or graphite bar 3 is located in the magnetic flux produced by the magnet. A plurality of similar interactive modules can be housed in a same receptacle as well.

As an example, the permanent magnet 2 is made of a compound sold on the market under the name of Alcomax III with the following composition by weight : 8.1 % aluminium, 13.5 % nickel, 24 % cobalt, 2.5 % copper, 0.5 % niobium and 51.4 % iron. The magnetisation of magnet is adapted to the appliance and can be chosen in the range of 500 to 3300 Gauss or so. The bar 3 consists for instance of Ellor 9 graphite, a fine-grained isotropic pure graphite yielding a maximum of 0.1 % ash on combustion and with a density of 1.80 g/cm3 and a resistivity of 1500 µΩcm. These parts can be embedded in a resin compound.

The device according to the invention has fixing means, e.g. a self adhesive means 5 at the bottom of the receptacle, for allowing the device to be placed on or inside a machine or apparatus emitting electromagnetic radiation. A number of such devices, preferably at least three units, will be placed on the machine or appliance. Examples of such sources of harmful radiation include devices with cathode-ray screens (television sets, the monitors of word-processing machines, personal computer displays), and motor vehicles. Figure 3 shows a simple diagram of a cathode-ray screen. On such a screen, three devices 1 are placed at the points of a triangle, as follows : one on top of the screen, the other two near the bottom of the screen.

During testing the applicant noticed that, thanks to the device according to this invention, the operators of electronic machines with cathode-ray screens could use the machines regularly and intensively without the recurrence of the complaints and disorders that usually follow after working on such machines.

Without giving a scientific explanation of the phenomenon that he has observed, the applicant hypothesizes that the devices as described in the foregoing and arranged according to this invention, the carbon or graphite is activated by the magnet field and acts as a sink for the exceeding radiation thereby to modify the surrounding electromagnetic field in which the operators of electronic machines are placed and thereby to counterbalance the harmful radiation such that the surrounding radiation do not subject these operators to physiological disorders.

The use of the device according to this invention is not limited to machines with cathode-ray screens, but includes all household appliances (microwave ovens, for example), computers, motor vehicles, and so on. When the machine is relatively large, it is preferable to use more than three units distributed over its entire surface.

A variation to the device of the invention is depicted in Fig. 4. This embodiment is for being used by a person for protecting said person from the surrounding electromagnetic radiation. In the example shown, the device contains preferably three interactive modules, each comprising two parts : a permanent magnet 2 and a bar 3 made of carbon and including magnesium these two parts being spaced apart such that the bar 3 is located in the magnetic flux generated by the magnet 2. The device of Fig. 4 has suspension means 6 for allowing the receptacle to be hung from a necklace. When so activated by the magnet flux, the bar 3 serves the same function as the carbon bar in the embodiment of Fig. 1, i.e. to counterbalance the electromagnetic radiation in excess, and in addition it acts as an activator for the physiological equilibrium of the person who is wearing the device as described.

Although there has been described hereinbefore two particular embodiments in accordance with the invention for the purpose of illustrating the manner in which the invention may be used to advantage, it will be appreciated that the invention is not limited thereto. Accordingly, various modifications, variations or equivalent arrangements which may occur to those skilled in the art should be considered to be within the scope of the invention as defined in the appended claims.

## Claims

1. A device to counterbalance the harmful effects of surrounding electromagnetic radiation, comprising a closed receptacle (1, 4) made of a non metallic material and at least one interactive module placed in the receptacle, each interactive module comprising two parts, a first of said parts consisting of a permanent magnet (2) and the second part consisting of a bar (3) made of carbon, natural or artificial graphite said two parts being spaced apart such that said bar (3) is located in the magnetic flux produced by said magnet (2).

2. A device according to claim 1, wherein the permanent magnet (3) is made of a compound having the following composition by weight : 8.1 % aluminium, 13.5 % nickel, 24 % cobalt, 2.5 % copper, 0.5 % niobium, and 51.4 % iron.

3. A device according to claims 1 or 2, wherein said (3) bar is composed of fine-grained isotropic pure graphite.

4. A device according to claim 3, wherein the pure graphite bar (3) is composed of an isotropic graphite yielding no more than 0.1 % ash upon combustion and with a density of 1.80 g/cm3 and a resistivity of 1500µΩcm.

5. A device according to any of claims 1 to 4, wherein the receptacle (1) has fixing means (5) for the fixation of the device onto a surface of or inside an apparatus.

6. A device according to claim 1 or 2, wherein said bar (3) further comprises magnesium.

7. A device according to any of claims 1, 2 and 6, wherein the receptacle (1) has suspension means (6) for allowing the receptacle to be hung from a necklace.

8. A device to counterbalance the harmful effects of machine-generated electromagnetic radiation, comprising several units according to any of claims 1 to 5, said units being distributed over a surface of or inside the machine.

## Patentansprüche

1. Vorrichtung zum Ausgleich schädlicher Auswirkungen von umgebender elektromagnetischer Strahlung mit einer geschlossenen Aufnahme (1, 4) aus nicht-metallischem Material und zumindest einem interaktiven Modul, das in der Aufnahme plaziert ist, wobei jedes interaktive Modul zwei Teile umfaßt, nämlich einen ersten Teil, der aus einem Permanentmagneten (2) besteht, und einen zweiten Teil, der aus einem Block (3) aus Kohlenstoff, natürlichem oder künstlichem Graphit besteht, welche zwei Teile so voneinander in Abstand stehen, daß der Block (3) innerhalb des durch den Magneten (2) erzeugten magnetischen Flusses angeordnet ist.

2. Vorrichtung nach Anspruch 1, wobei der Permanentmagnet (2) aus einer Verbindung besteht, die die folgende Zusammensetzung aufweist: 8,1 Gew.% Aluminium, 13,5 Gew.% Nickel, 24 Gew.% Kobalt, 2,5 Gew.% Kupfer, 0,5 Gew.% Niob und 51,4 Gew.% Eisen.

3. Vorrichtung nach Anspruch 1 oder 2, wobei der Block (3) aus feingemahlenem, isotropen Reingraphit besteht.

4. Vorrichtung nach Anspruch 3, wobei der Reingraphit-Block (3) aus einem isotropen Graphit besteht, der nach einer Verbrennung zu nicht mehr als 0,1 % Asche führt und der eine Dichte von 1,80 g/cm³ und einen Widerstand von 1500 µ Ω cm aufweist.

5. Vorrichtung nach einem der Ansprüche 1 bis 4, wobei die Aufnahme (1) eine Befestigungseinrichtung (5) zur Befestigung der Vorrichtung auf einer Fläche oder im Inneren eines Apparates aufweist.

6. Vorrichtung nach Anspruch 1 oder 2, wobei der Block (3) aus Magnesium besteht.

7. Vorrichtung nach einem der Ansprüche 1, 2 und 6, wobei die Aufnahme (1) eine Aufhängeeinrichtung (6) aufweist, die es erlaubt, die Aufnahme an einer Halskette aufzuhängen.

8. Vorrichtung zum Ausgleich der schädlichen Auswirkungen von Maschinen-erzeugter elektromagnetischer Strahlung, umfassend mehrere Einheiten nach einem der Ansprüche 1 bis 5, wobei die Einheiten über die Oberfläche oder den Innenraum der Maschine verteilt sind.

## Revendications

1. Dispositif pour contrebalancer les effets nuisibles de radiations électromagnétiques environnantes, comprenant un réceptacle fermé (1, 4) en matière non métallique et au moins un module interactif placé dans le réceptacle, chaque module interactif comprenant deux éléments, un premier élément consistant en un aimant permanent (2) et le second élément consistant en un barreau (3) en un matériau en carbone ou graphite naturel ou artificiel.

2. Dispositif suivant la revendication 1, caractérisé en ce que l'aimant permanent (2) est constitué d'un matériau composite comprenant, en poids, 8,1 % d'aluminium, 13,5 % de nickel, 24 % de cobalt, 2,5 % de cuivre, 0,5 % de niobium et 51,4 % de fer.

3. Dispositif suivant la revendication 1 ou 2, caractérisé en ce que ledit barreau (3) est constitué de graphite pur isotrope à grain fin.

4. Dispositif suivant la revendication 3, caractérisé en ce que le barreau de graphite pur (3) est constitué de graphite isotrope ayant un taux de cendres de 0,1 % maximum, une densité de 1,80 g/cm³ et une résistivité de 1500 µ.Ω.cm.

5. Dispositif suivant l'une quelconque des revendications 1 à 4, dans lequel le réceptacle (1) comporte des moyens de fixation (5) pour fixer le dispositif sur une surface ou à l'intérieur d'un appareil.

6. Dispositif suivant la revendication 1 ou 2, caractérisé en ce que le barreau (3) comprend en outre du magnésium.

7. Dispositif suivant l'une quelconque des revendications 1, 2 et 6, caractérisé en ce que le réceptacle (1) comporte un moyen de suspension (6) pour permettre de suspendre le réceptacle à un collier.

8. Dispositif pour contrebalancer les effets nuisibles des radiations électromagnétiques générées par un appareil, comprenant plusieurs modules suivant l'une quelconque des revendications 1 à 5, lesdits modules étant répartis sur une surface ou à l'intérieur de l'appareil.
